# FASCICULE DE BREVET EUROPEEN

(11) **EP 2 246 040 B1**
(45) Date de publication et mention de la délivrance du brevet: **02.03.2022**
(21) Numéro de dépôt: 10159678.1
(22) Date de dépôt: 12.04.2010
(51) Int. Cl.: A61K 8/73, A61K 8/81, A61K 8/84, A61K 8/88, A61K 8/89, A61K 8/92, A61Q 5/04, A61K 8/898

(54) **Procédé de mise en forme des cheveux au moyen d'une composition réductrice et d'un chauffage**
Verfahren zur Haarverformung, das die Anwendung einer reduzierenden Zusammensetzung und einen Erwärmungsschritt umfasst
Method for the shaping of hair including a step of applying a reducing composition and a heating step

(30) Priorité: 15.04.2009 FR 0952475
(43) Date de publication de la demande: 03.11.2010
(73) Titulaire: L'Oréal, 75008 Paris (FR)
(72) Inventeur: Dutheil-Gouret, Katia, 78370, Plaisir (FR); Pallanchard, Marion, 92120, Montrouge (FR); Livoreil, Aude, 75015, Paris (FR)
(74) Mandataire: L'Oreal

(56) Documents cités:
- EP-A- 1 312 650
- EP-A- 1 486 196
- EP-A- 1 935 396
- EP-A- 1 944 011
- EP-A- 2 011 478

## Description

La présente invention se rapporte à un procédé de traitement des fibres capillaires, ainsi qu'à l'utilisation dudit procédé.

Il est usuel, pour obtenir la déformation permanente des cheveux, de réaliser dans un premier temps l'ouverture des liaisons disulfures de la kératine (cystine) à l'aide d'une composition contenant un agent réducteur, puis après avoir de préférence rincé les cheveux, à reconstituer dans un second temps lesdites liaisons disulfures en appliquant sur les cheveux lissés ou préalablement mis sous tension par des moyens adéquats tels que les bigoudis, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Les compositions réductrices utilisables pour la mise en œuvre de la première étape de ces procédés contiennent généralement des composés thiolés, tels que l'acide thioglycolique, la cystéine, la cystéamine, l'acide thiolactique et le monothioglycolate de glycérol.

La concentration en agents réducteurs peut être très élevée, souvent jusqu'à 15% en poids de la composition réductrice.

Une telle technique ne donne pas entièrement satisfaction. En effet, cette technique est très efficace pour modifier la forme des cheveux, mais aussi très dégradante pour les fibres capillaires.

Il a en outre été proposé d'élever la température des cheveux, entre l'étape de réduction et l'étape de fixation, au moyen d'un fer chauffant.

Ainsi, la demande de brevet EP1584329 divulgue un procédé de traitement des fibres capillaires sans fixation comprenant les étapes suivantes :
- application sur les fibres capillaires d'une composition réductrice sans céramide, contenant au moins un agent réducteur, le ou les agents réducteurs étant choisis parmi les thiols et représentant moins de 3% en poids du poids total de la composition réductrice si la composition ne contient pas d'aminothiols, et moins de 5% si la composition contient au moins un aminothiol,
- élévation de la température des fibres capillaires, au moyen d'un fer chauffant, à une température au moins égale à 60°C, l'élévation de la température étant effectuée avant ou après le rinçage éventuel des fibres capillaires.

Cependant, une telle technique conduit à une mise en forme encore trop insuffisante, à des problèmes d'odeurs résiduelles et nécessite une application minutieuse du fer.

Le problème de la présente invention est donc de fournir un procédé de traitement des fibres capillaires qui remédie aux inconvénients de l'art antérieur.

En particulier, la présente invention a pour but de fournir un procédé de traitement des fibres capillaires permet de modifier la forme de la chevelure, de maîtriser le volume des cheveux, de réduire les frisotis et d'améliorer les performances cosmétiques des cheveux, notamment la douceur, la brillance et le démêlage en respectant mieux la couleur des cheveux teints.

On observe aussi que le procédé selon l'invention facilite les brushing ou lissage ultérieurs de la chevelure et limite les reprises de volume à 1

Un autre but de l'invention est de fournir un procédé de mise en forme des cheveux qui apporte encore plus de soin aux cheveux, sans laisser d'odeur résiduelle sur ces derniers après sa mise en œuvre.

La Demanderesse a trouvé qu'il était possible de remédier aux inconvénients de l'art antérieur et de remplir les objectifs précités, en mettant en œuvre un procédé de traitement des fibres capillaires sans fixation comprenant une étape d'application sur les fibres capillaires d'une composition réductrice, contenant au moins un agent réducteur associée à un polymère cationique, puis une étape d'élévation de la température des fibres capillaires, au moyen d'un fer chauffant, à une température au moins égale à 50°C, l'élévation de la température étant effectuée avant ou après le rinçage éventuel des fibres capillaires, ce procédé comprenant l'application d'une composition de soin, de préférence non-rincé, comprenant une ou plusieurs silicones aminées.

Ainsi, l'invention a pour objet un procédé de mise en forme des fibres capillaires comprenant les étapes suivantes :
(i) application sur les fibres capillaires d'une composition réductrice, comprenant un ou plusieurs polymère cationique, le rapport de la concentration en poids du réducteur sur la concentration en poids du polymère cationique étant compris entre 0,1 et 10, cette application étant suivie d'un rinçage,
(ii) application d'une composition de soin, de préférence non rincée, comprenant une ou plusieurs silicone aminée,
(iii) élévation de la température des fibres capillaires, à l'aide d'un moyen de chauffage, à une température comprise entre 50 et 280 °C, l'élévation de la température étant effectuée avant ou après le rinçage éventuel des fibres capillaires. 2. , l'étape (ii) étant réalisée après l'étape (i) et éventuellement avant l'étape (i), le procédé ne mettant en oeuvre aucune composition oxydante.

De préférence, le rapport de la concentration en poids du réducteur sur la concentration en poids du polymère cationique est compris entre 0,5 et 8, et plus préférentiellement entre 1 et 5.

L' expression *« procédé de traitement des fibres capillaires sans fixation »* décrit un procédé de traitement des fibres capillaires ne mettant pas en œuvre d'étape de fixation autre que la fixation à l'air. En particulier, cette expression signifie qu'on ne met pas en œuvre d'étape de fixation au moyen d'une composition oxydante comprenant de l'eau oxygénée à une teneur en eau oxygénée supérieure à 1 % en poids par rapport au poids total de la composition (soit 2 Volumes), laissée au contact des cheveux pendant plus de 5 minutes.

Selon l'invention, on ne met en œ uvre aucune composition oxydante.

Il est aussi décrit un de mise en œuvre du procédé dans lequel on met en œuvre une étape comprenant l'application d'une composition oxydante très faiblement concentrée en eau oxygénée, c'est-à-dire comprenant de l'eau oxygénée à une teneur inférieure à 1 % en poids par rapport au poids total de la composition (soit 2 Volumes) et on laisse agir cette composition pendant une durée inférieure à 5 minutes. Cette étape est utile pour neutraliser les odeurs.

### COMPOSITION REDUCTRICE

Avantageusement, on utilise un réducteur thiolé.

De préférence, le ou les thiols utilisés comme agents réducteurs dans la composition réductrice sont choisis parmi les aminothiols tels que la cystéïne et ses dérivés, comme la N-acétylcystéïne, la cystéamine et ses dérivés, de préférence ses dérivés acylés en C₁-C₄ tels que la N-acétyl cystéamine et la N-propionyl cystéamine,et les thiols non aminés tels que l'acide thiolactique et ses esters, comme le monothiolactate de glycérol, l'acide thioglycolique et ses esters, comme le monothioglycolate de glycérol ou de glycol, et le thioglycérol.

Lorsque le thiol possède au moins une fonction acide carboxylique, on peut le cas échéant utiliser ledit thiol sous forme d'un ou plusieurs de ses sels, comme les sels de métaux alcalins ou d'ammonium. On peut ainsi utiliser à titre de thiol du thioglycolate d'ammonium. Si le thiol possède un groupement amino on peut le cas échéant utiliser ledit thiol sous forme d'un ou plusieurs de ses sels comme les halogénures d'aminothiols. On peut ainsi utiliser à titre de thiol dans le cadre de la présente invention le chlorhydrate de L-cystéïne.

Comme aminothiols utilisables dans la composition réductrice utilisée selon l'invention, on peut encore citer les N-mercapto-alkylamides de sucres tels que le N-(mercapto-2-éthyl)gluconamide, la panthétéïne, les N-(mercaptoalkyl)ω-hydroxyalkylamides tels que ceux décrits dans la demande de brevet EP-A-354 835 et les N-mono- ou N,N-dialkylmercapto 4-butyramides tels que ceux décrits dans la demande de brevet EP-A-368763, les aminomercaptoalkylamides tels que ceux décrits dans la demande de brevet EP-A-432000 et les alkylaminomercaptoalkylamides tels que ceux décrits dans la demande de brevet EP-A-514282. Parmi les thiols non aminés utilisés dans l'invention on peut aussi citer le mélange de thioglycolate d'hydroxy-2 propyle (2/3) et de thioglycolate d'hydroxy-2 méthyl-1 éthyle (67/33) décrit dans la demande de brevet FR-A-2679448, l'acide ß-mercaptopropionique et ses dérivés, et l'acide thiomalique.

De préférence, selon l'invention, on utilise un réducteur thiolé, notamment l'acide thioglycolique ou la cystéine.

La concentration totale en réducteur et notemment en thiols des compositions réductrices de l'invention est de préférence comprise entre 0,1% et 5% par rapport au poids total de la composition.

### POLYMERE CATIONIQUE

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337354 et dans les brevets français FR-2270846, 2383660, 2598611, 2470596 et 2519863.

Les polymères cationiques préférés sont choisis parmi ceux qui contiennent des motifs comportant des groupements amine primaire, secondaire, tertiaire et/ou quaternaire pouvant, qui soit font partie de la chaîne principale polymère, soit sont portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2505348 ou 2542997. Parmi lesdits polymères, on peut citer :
(1) Les homopolymères ou copolymères dérivés d'esters ou d'amides acryliques ou méthacryliques, réticulés ou non, et comportant au moins un des motifs de formules (I), (II), (III) ou (IV) suivantes : 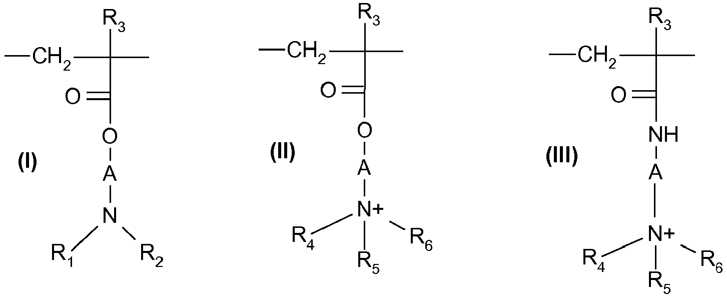 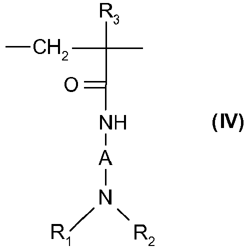 dans lesquelles
   R₃ désigne un atome d'hydrogène ou un radical CH₃;
   A représente un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 6 atomes de carbone ou un radical benzyle et de préférence un groupe alkyle ayant de 1 à 6 atomes de carbone ;
   R₁ et R₂, identiques ou différents, représentent hydrogène ou un groupe alkyle ayant de 1 à 6 atomes de carbone et de préférence méthyle ou éthyle ;
   X⁻ désigne un anion dérivé d'un acide minéral ou organique tel qu'un anion méthosulfate ou un halogénure tel que chlorure ou bromure.

Les polymères de la famille (1) peuvent contenir en outre un ou plusieurs motif(s) dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.

Ainsi, parmi ces polymères de la famille (1), on peut citer :
- les copolymères d'acrylamide et de diméthylaminoéthyl méthacrylate quaternisé au sulfate de diméthyle ou avec un halogénure de diméthyle, tel que celui vendu sous la dénomination HERCOFLOC par la société HERCULES,
- les copolymères d'acrylamide et de chlorure de méthacryloyloxyéthyl-triméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyl-oxyéthyltriméthylammonium vendu sous la dénomination RETEN par la société HERCULES,
- les copolymères vinylpyrrolidone/acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/vinylcaprolactame/ vinylpyrrolidone tel que le produit vendu sous la dénomination GAFFIX VC 713 par la société ISP,
- les copolymère vinylpyrrolidone/méthacrylamidopropyldimethylamine commercialisés notamment sous la dénomination STYLEZE CC 10 par ISP,
- les copolymères vinylpyrrolidone/méthacrylamide de diméthylaminopropyle quaternisés tel que le produit vendu sous la dénomination "GAFQUAT HS 100" par la société ISP,
- les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide / chlorure de méthacryloyloxyéthyl triméthylammonium (20/80 en poids) sous forme de dispersion contenant 50% en poids dudit copolymère dans de l'huile minérale. Cette dispersion est commercialisée sous le nom de " SALCARE^{®} SC 92 " par la Société CIBA. On peut également utiliser un homopolymère réticulé du chlorure de méthacryloyloxyéthyl triméthylammonium contenant environ 50% en poids de l'homopolymère dans de l'huile minérale ou dans un ester liquide. Ces dispersions sont commercialisées sous les noms de " SALCARE^{®} SC 95 " et " SALCARE^{®} SC 96 " par la Société CIBA.

(2) Les polysaccharides cationiques notamment choisis parmi :
a) les dérivés d'éthers de cellulose comportant des groupements ammonium quaternaire décrits dans le brevet français 1492597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
b) les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, ou de diméthyldiallylammonium.
   Les produits commercialisés répondant à cette définition sont plus particulièrement les produits vendus sous la dénomination "Celquat L 200" et "Celquat H 100" par la Société National Starch.
c) les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.

(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

(4) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.

(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylamino hydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polyamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347. Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique / époxypropyl / diéthylène-triamine.

(7) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 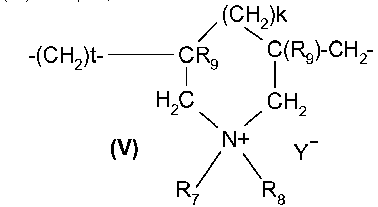 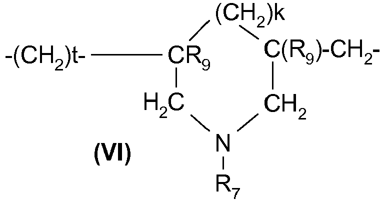 formules dans lesquelles
k et t sont égaux à 0 ou 1, la somme k + t étant égale à 1 ;
R₉ désigne un atome d'hydrogène ou un radical méthyle ;
R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ;
Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

Parmi les polymères définis ci-dessus, on peut citer plus particulièrement l'homopolymère de chlorure de diméthyldiallyl ammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

(8) Le polymère de diammonium quaternaire contenant des motifs récurrents répondant à la formule (VII) : 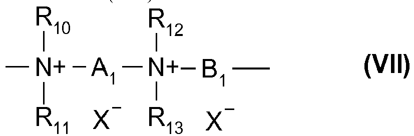 formule (VII) dans laquelle :
R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆, linéaire ou ramifié, substitué par un groupement nitrile, ester, acyle, amide ou -CO-O-R₁₄-D ou -CO-NH-R₁₄-D où R₁₄ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou
intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique ;
A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)ₙ-CO-D-OC-(CH₂)n-
dans lequel n est compris entre 1 et 100 et de préférence entre 1 et 50, et D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH- .

De préférence, X⁻ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante : 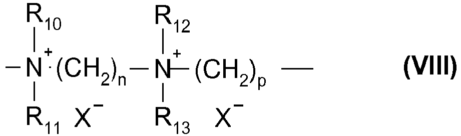 dans laquelle R₁₀, R₁₁, R₁₂ et R₁₃, identiques ou différents, désignent un radical alkyle ou hydroxyalkyle ayant de 1 à 4 atomes de carbone environ,
n et p sont des nombres entiers variant de 2 à 20 environ et,
X⁻ est un anion dérivé d'un acide minéral ou organique.

(9) Les polymères de polyammonium quaternaire constitués de motifs de formule (IX) : 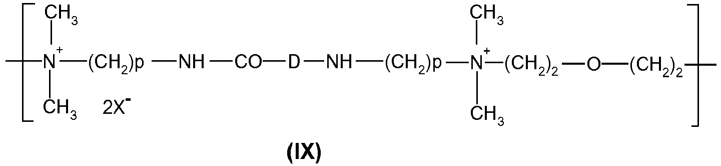 dans laquelle :
p désigne un nombre entier variant de 1 à 6 environ,
D peut être nul ou peut représenter un groupement
-(CH₂)ᵣ-CO- dans lequel r désigne un nombre égal à 4 ou à 7, et
X⁻ est un anion dérivé d'un acide minéral ou organique.

Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4 719 282.

Parmi ces polymères, on préfère ceux de masse moléculaire mesurée par RMN du Carbone 13 inférieure à 100000, et dans la formule de laquelle : p est égal à 3, et,
a) D représente un groupement -(CH₂)₄-CO- , X désigne un atome de chlore, la masse moléculaire mesurée par RMN du Carbone 13 (RMN¹³C) étant d'environ 5600 ; un polymère de ce type est proposé par la société MIRANOL sous le nom de MIRAPOL-AD1,
b) D représente un groupement -(CH₂)₇-CO- , X désigne un atome de chlore, la masse moléculaire mesurée par RMN du Carbone 13 ( RMN¹³C ) étant d'environ 8100; un polymère de ce type est proposé par la société MIRANOL sous le nom de MIRAPOL-AZ1,
c) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15,
d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).

Plus particulièrement encore, on préfère selon l'invention le polymère à motifs de formule (XI) dans laquelle p est égal à 3, D désigne la valeur zéro, X désigne un atome de chlore, la masse moléculaire mesurée par RMN du Carbone 13, ( RMN¹³C ) étant d'environ 25500.

(10) Les polymères quaternaires de vinylpyrrolidone et de vinylimidazole tels que par exemple les produits commercialisés sous les dénominations Luviquat FC 905, FC 550 et FC 370 par la société B.A.S.F. (11) Les polyamines comme le Polyquart H vendu par HENKEL, référencé sous le nom de " POLYETHYLENEGLYCOL (15) TALLOW POLYAMINE " dans le dictionnaire CTFA.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (7) et (8).

Le ou les polymère(s) cationique(s) selon l'invention se trouve(nt) en une quantité de 0,01 à 10% en poids, de préférence de 0,1 à 5% en poids, par rapport au poids total de la composition.

De préférence, les polymères cationiques sont choisis parmi le chlorure d'hexadiméthrine et les homo ou copolymères de chlorure de diméthyldiallylammonium.

Avantageusement, le pH de la composition réductrice est compris entre 4 et 12, de préférence entre 7 et 11.

Le réglage du pH de la composition peut être obtenu à l'aide d'un agent alcalin, tel que, par exemple, l'ammoniaque, les amines organiques comme la monoéthanolamine, la diéthanolamine, la triéthanolamine, la 1,3-propanediamine et le 2-amino-2-méthylpropanol, le 2-méthyl-1-aminopropanol, un carbonate ou bicarbonate alcalin ou d'ammonium, un carbonate organique tel que le carbonate de guanidine, un hydroxyde alcalin, tel que la soude, ou bien à l'aide d'un agent acidifiant tel que par exemple l'acide chlorhydrique, l'acide acétique, l'acide lactique, l'acide oxalique ou l'acide borique.

La composition réductrice comprend généralement un ou plusieurs solvants cosmétiquement acceptables, choisis de préférence parmi l'eau, les alcools en C₁-C₆, de préférence les alcanols tels que l'éthanol, le propanol et l'isopropanol, les polyols tels que le propylène glycol, le pentanediol et la glycérine et l'hexylène glycol, l'alcool benzylique, les éthers de polyols, les esters en C₂-C₆, la N-méthylpyrrolidone (NMP), et les cétones en C₃-C₆.

Dans le but d'améliorer les propriétés des compositions capillaires selon l'invention, la composition réductrice utilisée selon l'invention peut également comprendre un ou plusieurs additifs cosmétiques.

Ce ou ces additifs cosmétiques sont généralement choisis parmi les silicones volatiles ou non, linéaires ou cycliques, les polymères non-ioniques, anioniques ou amphotères, sous forme soluble ou insoluble telle que les latex, les peptides et leurs dérivés, les hydrolysats de protéines, les cires, les agents de gonflement et de pénétration ou permettant de renforcer l'efficacité du réducteur tels que le mélange SiO₂/polydiméthylsiloxane, le diméthylisosorbitol, l'urée et ses dérivés, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les agents opacifiants, les colorants, les filtres solaires, les vitamines ou provitamines, les acides gras, les alcools gras, les huiles minérales, végétales ou synthétiques, des particules minérales, ainsi que les parfums et les conservateurs, et leurs mélanges.

Le ou les actifs cosmétiques peuvent être choisis parmi les silicones.

Comme silicones utilisables comme actifs cosmétiques dans le procédé selon l'invention, on peut citer les polydiméthylsiloxanes, les polyorganosiloxanes quaternisés tels que ceux décrits dans la demande de brevet français FR 2535730, les polyorganosiloxanes à groupements aminoalkyles modifiés par des groupements alcoxycarbonylalkyles tels que ceux décrits dans le brevet US n° 4,749,732, des polyorganosiloxanes tels que le copolymère polydiméthylsiloxane- polyoxyalkyle du type diméthicone copolyol, un polydiméthylsiloxane à groupements terminaux stéaroxy (stéaroxydiméthicone), un copolymère polydiméthylsiloxane- dialkylammonium acétate ou un copolymère polydiméthyl-siloxane polyalkylbétaïne décrits dans le brevet britannique GB 2197352, des polysiloxanes organo modifiés par des groupements mercapto ou mercaptoalkyles tels que ceux décrits dans le brevet français FR 1530369 et dans la demande de brevet européen EP 295780.

Par ailleurs, le ou les actifs cosmétiques peuvent être également choisis parmi les acides gras et les alcools gras.

Comme acides gras utilisables comme actifs dans le procédé selon l'invention, on peut notamment citer les acides carboxyliques en C₈-C₃₀, tels que l'acide palmitique, l'acide oléique, l'acide linoléique, l'acide myristique, l'acide stéarique, l'acide laurique, et leurs mélanges.

Comme alcools gras utilisables dans la présente invention, on peut notamment citer les alcools en C₈-C₃₀ comme, par exemple, les alcools palmitylique, oléylique, linoléylique, myristylique, stéarylique et laurylique ou leurs mélanges.

La composition réductrice utilisée dans le procédé selon l'invention peut se présenter sous la forme d'une lotion, épaissie ou non, d'une crème, d'un gel, ou d'une mousse.

Après l'application de la composition réductrice, on peut laisser poser ladite composition, généralement pendant 5 à 60 minutes, de préférence 5 à 30 minutes, éventuellement sous casque.

### COMPOSITION DE SOIN (ii)

Dans tout ce qui suit ou qui précède, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements amides, les radicaux acyloxy ou acyloxyalkyls, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon l'invention, on désigne par silicone aminée utilisée dans l'étape (ii), toute silicone comportant au moins une amine primaire, secondaire, tertiaire ou un groupement ammonium quaternaire. On peut ainsi citer :
(a) les polysiloxanes répondant à la formule : 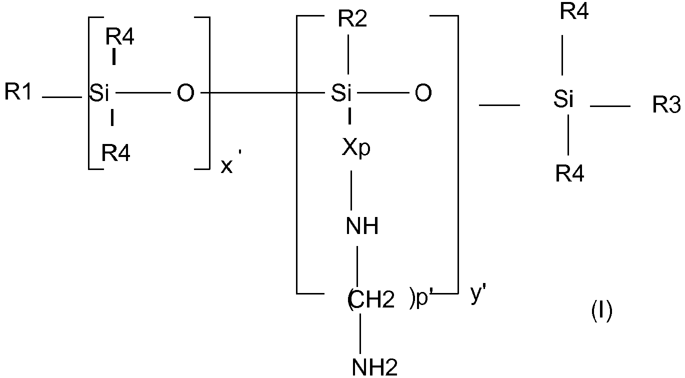
   dans laquelle x' et y' sont des nombres entiers dépendant du poids moléculaire, généralement tels que ledit poids moléculaire moyen en poids est compris entre 5000 et 500000 environ ;
   R₁, R₂, R₃ identiques ou différents désignent un radical hydroxyle, un radical alkyle en C₁ à C₄, un radical alcoxy en C₁ à C₄, phényle ;
   X désigne un radical alkylène en C₁-C₄, ramifié ou non ;
   R₄ désigne un radical alkyle en C₁-C₄ ou phényle ;
   p, p' désignent indépendamment l'un de l'autre un nombre entier variant de 1 à 10.
   De préférence, alkyle désigne méthyl et alcoxy désigne méthoxy.
   De préférence, p=3, p'=2 et R4 désigne méthyl et X désigne méthylène.
   Parmi ces polymères, on peut citer les composés désignés par l'appellation «amodiméthicone» et «triméthylsilylaminodiméthicone» dans le dictionnaire CTFA.
(b) les silicones aminées répondant à la formule : 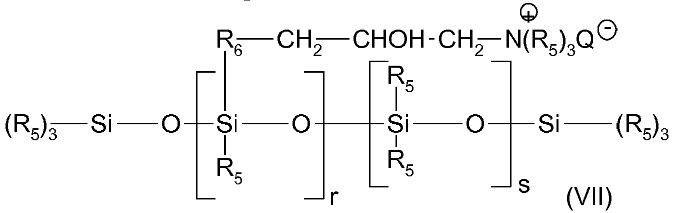 dans laquelle
   R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   Q⁻ est un anion tel qu'un ion halogénure, notamment chlorure ou un sel d'acide organique (acétate ...);
   r représente une valeur statistique moyenne de 2 à 20 et en particulier de 2 à 8 ;
   s représente une valeur statistique moyenne de 20 à 200 et en particulier de 20 à 50.
   De telles silicones aminées sont décrites plus particulièrement dans le brevet US 4185087.
   Une silicone entrant dans cette classe est la silicone commercialisée par la société Union Carbide sous la dénomination "Ucar Silicone ALE 56.
c) les silicones ammonium quaternaire de formule : 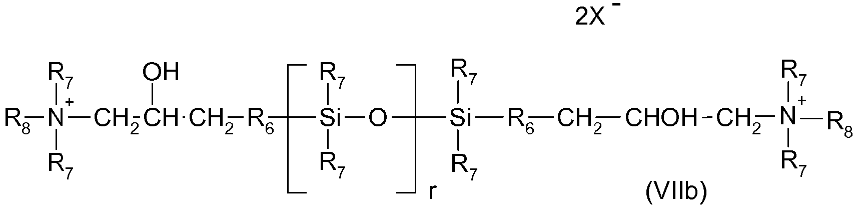 dans laquelle
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100 ;

Ces silicones sont par exemple décrites dans la demande EP-A-0530974.

Selon l'invention, on préfère utiliser une silicone aminée:
- dont l'indice d'amine est supérieur à 0,15 meq par gramme,
- possédant des extréminés hydroxy et/ou alcoxy.

A titre d'exemple, on peut citer, comme microémulsion, contenant une telle silicone, le produit commercialisé par la Société WACKER sous le nom de WACKER-BELSIL ADM LOG 1.

Les silicones aminées peuvent se présenter sous la forme de micrémulsion.

Avantageusement, la concentration en silicone aminée dans la composition de pré-traitement ou de post-traitement est comprise entre 0,05 et 10 % en poids par rapport au poids total de cette composition, et plus avantageusement entre 0,1,et 7 %.

Les compositions de soin contenant les silicones aminées peuvent contenir des actifs hydrosolubles ou liposolubles, ayant une activité cosmétique. On peut citer, à titre d'exemples d'actif, les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines telles que le panthénol, le palmitate de vitamine A, la niacinamide, l'ergocalciférol, les anti oxydants, les huiles essentielles, les humectants, les filtres solaires siliconés ou non, des agents conservateurs, des séquestrants, des agents nacrants, des pigments, des agents hydratants, des agents antipelliculaires, des agents antiséborrhéiques, des plastifiants, des hydroxyacides, des électrolytes et des parfums. Les compositions de soin contenant les silicones aminées peuvent contenir un ou plusieurs des additifs énumérés pour les compositions réductrices.

De préférence, le pH de la composition de silicone aminée est compris de préférence entre 2 et 10, et plus préférentiellement entre 3 et 9.

De telles compositions de soin sont décrites dans EP1 247 518.

### ETAPE DE CHAUFFAGE

Comme expliqué précédemment, le procédé selon l'invention comprend une étape d'élévation de la température des fibres capillaires, à l'aide d'un moyen de chauffage.

Comme moyen de chauffage, on peut utiliser un casque, un sèche-cheveux, un fer avec ou sans ajout de vapeur, notamment tel que décrit dans FR 2921805, un dispositif infrarouge.

L'étape de chauffage peut être précédée d'une étape de pré-séchage, à l'aide d'un ou plusieurs moyens de chauffage..

Au sens de la présente invention, on entend par fer un dispositif de chauffage des fibres capillaires par contact.

L'extrémité du fer venant en contact avec les cheveux peut avoir différentes formes. Elle peut notamment présenter une surface plane ; on parle dans ce cas de fer plat. Elle peut également présenter une surface arrondie ; on parle dans ce cas de fer rond.

L'application du fer peut se faire par touches séparées successives de quelques secondes, ou par déplacement ou glissement progressif le long des mèches.

A titre d'exemple de fer utilisable dans le procédé selon l'invention, on peut citer tous types de fer plats ou ronds et, en particulier, de manière non limitative, ceux décrits dans les brevets US 4103145, US 4308878, US 5983903, US 5957140, US 5494058 et US 5046516.

De préférence, la température des fibres capillaires est élevée jusqu'à une température comprise entre 60°C et 250°C, mieux entre 120°C et 220°C.

Dans le cas de l'utilisation d'un casque ou d'un sèche-cheveux, le temps de séchage peut varier de 1 à 90 minutes.

L'ordre des étapes (i), (ii) et (iii) est indifférent. De préférence, le procédé de l'invention peut être illustré par les variantes suivantes :
Variante 1 : étape i, puis étape ii puis étape iii, (selon l'invention),
Variante 2 : étape ii, puis étape i puis étape iii, (pas selon l'invention),
Variante 3 : étape ii, puis étape i puis étape ii puis étape iii. (selon l'invention).

L'étape ii peut éventuellement être suivie d'un rinçage ou d'un shampooing, de préférence, l'étape ii est non rincée. Si l'étape ii est suivie d'un rinçage, le temps de pose avant le rinçage est compris entre 30 secondes et 10 minutes.

### ETAPES SUPPLEMENTAIRES EVENTUELLES

### Application d'une composition de soin (iv)

Le procédé conforme à l'invention peut comprendre, en outre, l'application d'une composition de soin (iv) différent de la composition de soin (ii). Cette composition comprend au moins un corps gras siliconé ou non.

Les corps gras utilisables dans les compositions de soin (iv) selon la présente invention sont toutes les huiles, cires, résines siliconées ou non, organiques ou minérales, naturelles ou synthétiques.

Une huile, au sens de la présente invention, est un composé lipophile, liquide à température ambiante (environ 25 °C), à changement d'état solide/liquide réversible. Les huiles animales et végétales comprennent comme constituants essentiels des triesters du propane-1,2,3-triol.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope.

### CORPS GRAS NON SILICONES

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arana, de tournesol, de ricin, d'avocat, les triglycérides des acides caprylique/caprique comme par exemple ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R⁶COOR⁷ et R⁶OR⁷ dans laquelle R⁶ représente le reste d'un acide gras comportant de 8 à 29 atomes de carbone, et R⁷ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononaoate de diéthylèneglycol ; et les esters de pendaérythritol comme le tétraisostéarate de pentaérythrityle ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras fluides ayant de 8 à 26 atomes de carbone, comme l'octyldodécanol, le 2-butyloctanol, l'alcool oléique, l'alcool linoléique ou l'alcool linolénique ;
- les alcools gras alcoxylés et notamment éthoxylés tels que l'oleth-12 ;
- les huiles fluorées partiellement hydrocarbonées comme celles décrites dans le document JP-A-2 295912. Comme huiles fluorées, on peut citer aussi le perfluorométhylcyclopentane et le perfluoro-1,3- diméthylcyclohexane, par exemple vendus sous les dénominations de « FLUTEC PC1^{®} » et « FLUTEC PC3^{®} » par la Société BNFL Fluorochemicals ; le perfluoro-1,2-diméthylcyclobutane ; les perfluoralcanes tels que le dodécafluoropentane et le tétradécafluorohexane, par exemple vendus sous les dénominations de « PF 5050^{®} » et « PF 5060^{®} » par la Société 3M, ou encore le bromoperfluorooctyle par exemple vendu sous la dénomination « FORALKYL^{®} » par la Société Atochem ; le nanofluorométhoxybutane par exemple vendu sous la dénomination « MSX 4518^{®} » par la Société 3M et le nanofluoroéthoxyisobutane ; les dérivés de perfluoromorpholine, tels que la 4-trifluorométhyl perfluoromorpholine par exemple vendue sous la dénomination « PF 5052^{®} » par la Société 3M.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les cires animales et végétales comprennent comme constituants essentiels des esters d'acides carboxyliques et d'alcools à longues chaînes. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que les cires de tournesol, de riz, de pomme, la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines, la cérésine ou l'ozokérite ; les cires synthétiques telles que les cires de polyéthylènes, les cires de Fischer-Tropsch, et leurs mélanges.

### CORPS GRAS SILICONE

On peut également utiliser, dans la composition de soin (iv) selon l'invention, une silicone en tant que corps gras.

Les silicones utilisables dans les compositions selon la présente invention peuvent être linéaires, cyclique, ramifiées ou non ramifiées, volatiles ou non volatiles. Elles peuvent se présenter sous forme d'huiles, de résines ou de gommes, elles peuvent en particulier être des polyorganosiloxanes insolubles dans le milieu cosmétiquement acceptable.

Les organopolysiloxanes sont définis plus en détail dans l'ouvrage de Walter NOLL "Chemistry and Technology of Silicones" (1968) Academic Press. Ils peuvent être volatils ou non volatils.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.

On peut également citer les cyclocopolymères du type diméthylsiloxane/méthylalkylsiloxane, tel que la "SILICONE VOLATILE FZ 3109" commercialisée par la société UNION CARBIDE, de structure chimique :

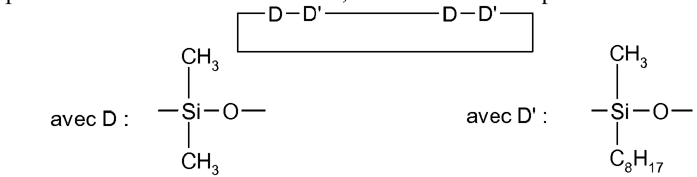

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10-6m2/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicone, les polyorganosiloxanes modifiés par des groupements organofonctionnels ainsi que leurs mélanges.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C6-C24 tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C12)-méthicone-copolyol commercialisée par la société DOW CORNING sous la dénomination Q2 5200 ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- Les huiles de silicone utilisables dans les compositions selon l'invention sont des polyméthylsiloxanes volatiles ou non, à chaîne siliconée linéaire ou cyclique, liquides ou pâteuse à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthyl-phénylsiloxanes ; et leurs mélanges.

Les gommes de silicone utilisables dans les compositions selon l'invention sont des polydiorganosiloxanes de masse moléculaire élevée, comprise entre 200 000 et 2 000 000, utilisées seules ou en mélange dans un solvant choisi parmi les silicones volatiles, les huiles polydiméthylsiloxanes, les huiles polyméthylphénylsiloxanes, les huiles polydiphényldiméthylsiloxanes, les isoparaffines, le chlorure de méthylène, le pentane, les hydrocarbures ou leurs mélanges.

On utilise de préférence une gomme de silicone de poids moléculaire inférieur à 1 500 000. Les gommes de silicone sont par exemple des polydiméthylsiloxanes, des polyphénylméthylsiloxanes, des poly(diphénylsiloxane diméthylsiloxanes), des poly(diméthylsiloxaneméthylvinylsiloxanes), des poly(diméthylsiloxanephénylméthylsiloxanes), des poly(diphénylsiloxane diméthylsiloxaneméthylvinylsiloxanes).

Ces gommes de silicones peuvent être terminées en bout de chaîne par des groupements triméthylsilyls ou diméthylhydroxysilyls.

Les résines de silicone utilisables dans les compositions selon l'invention sont des systèmes siloxaniques réticulés renfermant les unités R₂SiO_{2/2}, RSiO_{3/2}, SiO_{4/2}, dans lesquelles R représente un groupe hydrocarbonés possédant de 1 à 6 atomes de carbone ou un groupe phényle. Parmi ces produits, ceux particulièrement préférés sont ceux dans lesquelles R désigne un radical alkyl inférieur (C₁-C₆) ou un radical phényle.

La concentration en corps gras siliconés ou non, utilisés dans les compositions (iv) selon la présente invention, est comprise entre 0,01 et 20% et de préférence entre 0,05 et 10% en poids par rapport au poids total de la composition.

La composition (iv) peut contenir tous les ingrédients additionnels de la composition ii.

De préférence, la composition de soin iv est appliquée avant l'étape i et est éventuellement rincée. Si elle est rincée, son temps de pause est compris entre 30 secondes et 10 minutes.

La présente invention est illustrée par les exemples suivants. Les teneurs sont indiquées en matière active.

### Exemples

On met en œuvre le procédé de traitement des fibres capillaires selon l'invention en utilisant une composition réductrice et une composition de soin.

### Composition réductrice B

| | |
|---|---|
| Cysteine | 4 g |
| Hydroxypropylguar | 0.5 g |
| Mexomere PO | 2.5g |
| Monoéthanolamine | qsp pH 9,2 |
| Eau | qsp 100g |

| Composition de soin D | |
|---|---|
| Cetearyl alcohol | 1.5g |
| Steareth-20 | 0.5 |
| WACKER-BELSIL ADM LOG 1 | 13.5 |
| Eau | qsp 100g |

On met en œuvre le procédé de traitement des fibres capillaires selon l'invention en utilisant, comme moyen d'élévation de la température, soit un sèche-cheveux, soit un fer.

### 1) Exemple de procédé avec sèche-cheveux

Ce procédé présente l'avantage d'être rapide et peu technique, ce qui permet à la modèle de se l'appliquer.

La chevelure (cheveux naturels moyennement ondulés) est lavée à l'aide d'un shampooing standard puis essorée.

Le soin D est appliqué immédiatement sur les pointes, sans rinçage, à température ambiante (22°C).

La composition réductrice B est appliquée à la suite sur l'ensemble de la chevelure, à la main ou au pinceau. Un temps de pause de 20 min, à température ambiante, est respecté, puis la chevelure est rincée à l'eau abondamment.

Le soin D est appliqué sur l'ensemble de la chevelure et la chevelure est pré séchée au sèche-cheveux. On procède ensuite à un brushing soigné de l'ensemble de la chevelure, toujours avec un sèche-cheveux et une brosse, à une température d'environ 60°C.

Au final, la chevelure est lisse, sans frisottis et les fibres sont faciles à coiffer et à démêler. Les cheveux ne sont pas dégradés.

Au cours des jours qui suivent, et ce jusqu'à environ 8 shampooings, les cheveux restent plus faciles à coiffer, avec moins de frisottis, des boucles plus détendues. Le brushing est rapide et facile.

### 2) Exemple de procédé avec fer

Ce procédé est plus technique que le précédent, mais il présente l'avantage d'un résultat plus lisse et un peu plus rémanent, tout en étant réversible.

La chevelure (cheveux naturels moyennement ondulés) est lavée à l'aide d'un shampooing standard puis essorée.

Le soin D est appliqué immédiatement sur les pointes, sans rinçage, à température ambiante (22°C).

La composition réductrice B est appliquée à la suite sur l'ensemble de la chevelure, à la main ou au pinceau. Un temps de pause de 20 min, à température ambiante, est respecté, puis la chevelure est rincée à l'eau abondamment.

Le soin D est appliqué sur l'ensemble de la chevelure et la chevelure est pré séchée au sèche-cheveux. On procède ensuite à un lissage soigné de l'ensemble de la chevelure avec un fer plat dont la température est de 180°C.

La chevelure est lisse, sans frisottis et les fibres sont faciles à coiffer et à démêler.

Au cours des jours qui suivant, et ce jusqu'à environ 10 shampooings, les cheveux restent plus faciles à coiffer, avec moins de frisottis, des boucles plus détendues. Le brushing quotidien est rapide et facile.

Les cheveux ne sont pas dégradés et l'odeur résiduelle est faible.

## Revendications

1. Procédé de mise en forme des fibres capillaires comprenant les étapes suivantes :
(i) application sur les fibres capillaires d'une composition réductrice, comprenant un ou plusieurs polymère cationique, le rapport de la concentration en poids du réducteur sur la concentration en poids du polymère cationique étant compris entre 0,1 et 10, cette application étant suivie d'un rinçage,
(ii) application d'une composition de soin, de préférence non rincée, comprenant une ou plusieurs silicone aminée,
(iii) élévation de la température des fibres capillaires, à l'aide d'un moyen de chauffage, à une température comprise entre 50 et 280 °C, l'élévation de la température étant effectuée avant ou après le rinçage éventuel des fibres capillaires,
l'étape (ii) étant réalisée après l'étape (i) et éventuellement avant l'étape (i),
le procédé ne mettant en œuvre aucune composition oxydante.

2. Procédé selon la revendication 1, **caractérisé par le fait que** le rapport de la concentration en poids du réducteur sur la concentration en poids du polymère cationique est compris entre 0,5 et 8, et plus préférentiellement entre 1 et 5.

3. Procédé selon l'une quelconque des revendications 1 ou 2, **caractérisé par le fait que** le polymère cationique utilisé dans l'étape (i), est choisi parmi les cyclopolymères d'alkyl diallyl amine ou de dialkyl diallyl ammonium tels que les homopolymères ou copolymères comportant comme constituant principal de la chaîne des motifs répondant aux formules (V) ou (VI) : 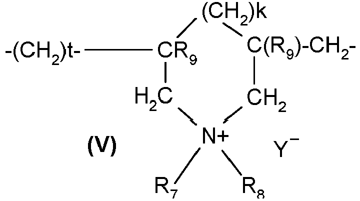 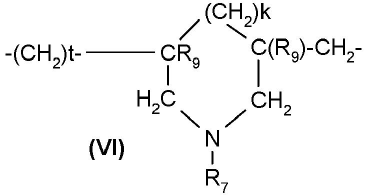 formules dans lesquelles
k et t sont égaux à 0 ou 1, la somme k + t étant égale à 1 ;
R₉ désigne un atome d'hydrogène ou un radical méthyle ;
R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ;
Y⁻ est un anion tel que bromure, chlorure, acétate, borate, citrate, tartrate, bisulfate, bisulfite, sulfate, phosphate.

4. Procédé selon l'une quelconque des revendications 1 ou 2, **caractérisé par le fait que** le polymère cationique utilisé dans l'étape (i) est choisi parmi les polymères de diammonium quaternaire contenant des motifs récurrents répondant à la formule (VII) : 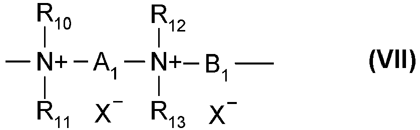 formule (VII) dans laquelle :
R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆, linéaire ou ramifié, substitué par un groupement nitrile, ester, acyle, amide ou -CO-O-R₁₄-D ou -CO-NH-R₁₄-D où R₁₄ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique ;
A1, Rio et R₁₂ peuvent former avec les deux atomes d'azote auxquels ils sont rattachés un cycle pipérazinique ; en outre si A1 désigne un radical alkylène ou
hydroxyalkylène linéaire ou ramifié, saturé ou insaturé, B1 peut également désigner un groupement
-(CH₂)ₙ-CO-D-OC-(CH₂)n-
dans lequel n est compris entre 1 et 100 et de préférence entre 1 et 50, et D désigne :
a) un reste de glycol de formule : -O-Z-O-, où Z désigne un radical hydrocarboné linéaire ou ramifié ou un groupement répondant à l'une des formules suivantes :
-(CH₂-CH₂-O)ₓ-CH₂-CH₂-
-[CH₂-CH(CH₃)-O]_{y}-CH₂-CH(CH₃)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH₂-CH₂-S-S-CH₂-CH₂- ;
d) un groupement uréylène de formule : -NH-CO-NH- .

5. Procédé selon l'une quelconque des revendications précédentes, **caractérisé par le fait que** la concentration en réducteur dans la composition de l'étape (i) est comprise entre 0,1% et 5% par rapport au poids total de la composition.

6. Procédé selon l'une quelconque des revendications précédentes, **caractérisé par le fait que** la concentration en polymère(s) cationique(s) dans la composition de l'étape i, va de 0,01 à 10 % en poids, de préférence de 0,1 à 5 % en poids, par rapport au poids total de la composition.

7. Procédé selon l'une quelconque des revendications précédentes **caractérisé en ce que** la température des fibres capillaires est élevée jusqu'à une température comprise entre 80°C et 220°C, dans l'étape (iii).

8. Procédé selon l'une quelconque des revendications précédentes **caractérisé en ce qu'**on utilise un réducteur thiolé, dans l'étape (i).

9. Procédé selon l'une quelconque des revendications précédentes, **caractérisé en ce qu'**on utilise, comme silicone aminée dans la composition de soin (ii), une silicone aminée répondant à la formule I : 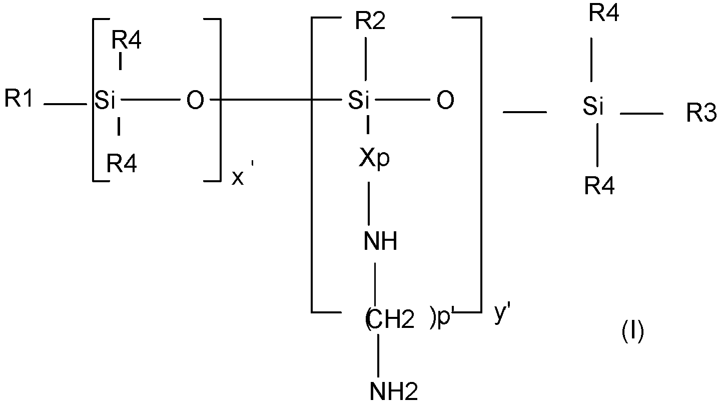
dans laquelle x' et y' sont des nombres entiers dépendant du poids moléculaire, généralement tels que ledit poids moléculaire moyen en poids est compris entre 5 000 et 500 000 environ ;
R1, R2, R3 identiques ou différents désignent un radical hydroxyle, un radical alkyle en C1 à C4, un radical alcoxy en C1 à C4, phényle ;
X désigne un radical alkylène en C1-C4, ramifié ou non ;
R4 désigne un radical alkyle en C1-C4 ou phényle ;
p, p' désignent indépendamment l'un de l'autre un nombre entier variant de 1 à 10.

10. Procédé selon l'une quelconque des revendications précédentes, **caractérisé par le fait que** le procédé comprend, en outre, l'application d'une composition de soin (iv) différente de la composition de soin (ii) comprenant la silicone aminée, cette composition comprenant au moins un corps gras siliconé ou non

## Patentansprüche

1. Verfahren zur Gestaltung von Haarfasern, das folgende Schritte umfasst:
(i) Aufbringen einer reduzierenden Zusammensetzung, die ein oder mehrere kationische Polymere umfasst, auf die Haarfasern, wobei das Verhältnis der Gewichtskonzentration des Reduktionsmittels zur Gewichtskonzentration des kationischen Polymers zwischen 0,1 und 10 liegt, wobei auf dieses Aufbringen eine Spülung folgt,
(ii) Aufbringen einer Pflegezusammensetzung, vorzugsweise einer Pflegezusammensetzung, die nicht ausgespült wird, umfassend ein oder mehrere Aminosilikone,
(iii) Erhöhen der Temperatur der Haarfasern mit einem Erhitzungsmittel auf eine Temperatur zwischen 50 und 280 °C, wobei das Erhöhen der Temperatur vor oder nach dem fakultativen Spülen der Haarfasern durchgeführt wird,
wobei der Schritt (ii) nach dem Schritt (i) und gegebenenfalls vor dem Schritt (i) durchgeführt wird,
wobei bei dem Verfahren keine oxidierende Zusammensetzung eingesetzt wird.

2. Verfahren nach Anspruch 1, **dadurch gekennzeichnet, dass** das Verhältnis der Gewichtskonzentration des Reduktionsmittels zur Gewichtskonzentration des kationischen Polymers zwischen 0,5 und 8 und weiter bevorzugt zwischen 1 und 5 liegt.

3. Verfahren nach Anspruch 1 oder 2, **dadurch gekennzeichnet, dass** das in Schritt (i) verwendete kationische Polymer aus Alkyldiallylamin- oder Dialkyldiallylammonium-Cyclopolymeren wie den Homopolymeren oder Copolymeren, die als Hauptbestandteil der Kette Einheiten der Formel (V) oder (VI) : 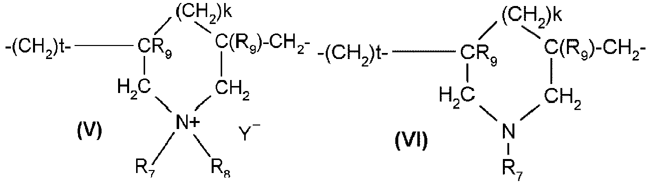
umfassen, ausgewählt ist, wobei in den Formeln k und t gleich 0 oder 1 sind, wobei die Summe k + t gleich 1 ist;
R₉ für ein Wasserstoffatom oder einen Methylrest steht;
R₇ und R₈ unabhängig voneinander eine Alkylgruppe mit 1 bis 6 Kohlenstoffatomen, eine Hydroxyalkylgruppe, in der die Alkylgruppe vorzugsweise 1 bis 5 Kohlenstoffatome aufweist, eine niedere (C₁-C₄) Amidoalkylgruppe bedeuten oder R₇ und R₈ zusammen mit dem Stickstoffatom, an das sie gebunden sind, heterocyclische Gruppen wie Piperidinyl oder Morpholinyl bedeuten können; R₇ und R₈ unabhängig voneinander vorzugsweise eine Alkylgruppe mit 1 bis 4 Kohlenstoffatomen bedeuten; Y⁻ für ein Anion wie Bromid, Chlorid, Acetat, Borat, Citrat, Tartrat, Bisulfat, Bisulfit, Sulfat oder Phosphat steht.

4. Verfahren nach Anspruch 1 oder 2, **dadurch gekennzeichnet, dass** das in Schritt (i) verwendete kationische Polymer aus quaternären Diammoniumpolymeren mit Wiederholungseinheiten der Formel (VII): 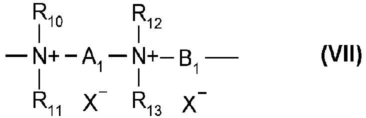 ausgewählt ist, wobei in Formel (VII):
R₁₀, R₁₁, R₁₂ und R₁₃ gleich oder verschieden sind und für aliphatische, alicyclische oder arylaliphatische Reste mit 1 bis 6 Kohlenstoffatomen oder niedere hydroxyalkylaliphatische Reste stehen oder auch R₁₀, R₁₁, R₁₂ und R₁₃ zusammen oder separat mit den Stickstoffatomen, an die sie gebunden sind, Heterocyclen, die gegebenenfalls neben dem Stickstoff ein zweites Heteroatom enthalten, bilden oder auch R₁₀, R₁₁, R₁₂ und R₁₃ für einen linearen oder verzweigten C₁-C₆-Alkylrest, der durch eine Nitril-, Ester-, Acyl-, Amid- oder -CO-O-R₁₄D- oder -CO-NH-R₁₄-D-Gruppe substituiert ist, stehen, wobei R₁₄ für ein Alkylen steht und D für eine quaternäre Ammoniumgruppe steht;
A₁ und B₁ für Polymethylengruppen mit 2 bis 20 Kohlenstoffatomen stehen, die linear oder verzweigt und gesättigt oder ungesättigt sind und an die Hauptkette gebunden oder in die Hauptkette eingeschoben einen oder mehrere aromatische Ringe oder ein oder mehrere Sauerstoff- oder Schwefelatome oder Sulfoxid-, Sulfon-, Disulfid-, Amino-, Alkylamino-, Hydroxyl-, quaternäre Ammonium-, Ureido-, Amid- oder Estergruppen enthalten können, und
X⁻ ein Anion, das sich von einer Mineralsäure oder organischen Säure ableitet, bedeutet;
A₁, R₁₀ und R₁₂ mit den beiden Stickstoffatomen, an die sie gebunden sind, einen Piperazinring bilden können; außerdem dann, wenn A₁ für einen linearen oder verzweigten, gesättigten oder ungesättigten Alkylen- oder Hydroxyalkylenrest steht, B₁ auch eine Gruppe
-(CH₂)ₙ-CO-D-OC-(CH₂)ₙ-
bedeuten kann, wobei n zwischen 1 und 100 und vorzugsweise zwischen 1 und 50 liegt und D Folgendes bedeutet:
a) einen Glykolrest der Formel: -O-Z-O-, wobei Z einen linearen oder verzweigten Kohlenwasserstoffrest oder eine Gruppe, die einer der folgenden Formeln entspricht, bedeutet:
-(CH₂-CH₂-O)ₓ-CH₂-CH₂-
- [CH₂-CH(CH₃)-O]_{y}-CH₂-CH(CH₃)-,
wobei x und y eine ganze Zahl von 1 bis 4, die einen definierten und einzigartigen Polymerisationsgrad wiedergibt, oder eine beliebige Zahl von 1 bis 4, die einen durchschnittlichen Polymerisationsgrad wiedergibt, bedeuten;
b) einen bissekundären Diaminrest wie ein Piperazinderivat;
c) einen bisprimären Diaminrest der Formel: -NH-Y-NH-, wobei Y einen linearen oder verzweigten Kohlenwasserstoffrest oder auch den zweiwertigen Rest
-CH₂-CH₂-S-S-CH₂-CH₂-
bedeutet;
d) eine Ureylengruppe der Formel: -NH-CO-NH-.

5. Verfahren nach einem der vorhergehenden Ansprüche, **dadurch gekennzeichnet, dass** die Konzentration an Reduktionsmittel in der Zusammensetzung von Schritt (i) im Bereich von 0,1 bis 5 Gew.-%, bezogen auf das Gesamtgewicht der Zusammensetzung, liegt.

6. Verfahren nach einem der vorhergehenden Ansprüche, **dadurch gekennzeichnet, dass** die Konzentration an kationischem Polymer bzw. kationischen Polymeren in der Zusammensetzung von Schritt i im Bereich von 0,01 bis 10 Gew.-%, vorzugsweise 0,1 bis 5 Gew.-%, bezogen auf das Gesamtgewicht der Zusammensetzung, liegt.

7. Verfahren nach einem der vorhergehenden Ansprüche, **dadurch gekennzeichnet, dass** die Temperatur der Haarfasern in Schritt (iii) auf eine Temperatur zwischen 80 °C und 220 °C erhöht wird.

8. Verfahren nach einem der vorhergehenden Ansprüche, **dadurch gekennzeichnet, dass** man in Schritt (i) ein Thiol-Reduktionsmittel verwendet.

9. Verfahren nach einem der vorhergehenden Ansprüche, **dadurch gekennzeichnet, dass** man als Aminosilikon in der Pflegezusammensetzung (ii) ein Aminosilikon der Formel I verwendet: 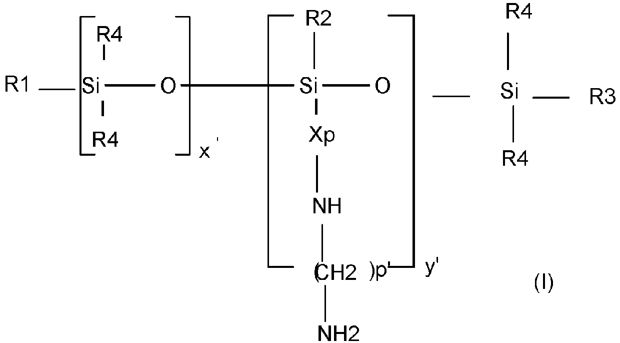
in der x' und y' vom Molekulargewicht abhängende ganze Zahlen sind, im Allgemeinen derart, dass das gewichtsmittlere Molekulargewicht zwischen ungefähr 5000 und 500.000 liegt;
R1, R2 und R3 gleich oder verschieden sind und einen Hydroxylrest, einen C1- bis C4-Alkylrest, einen C1-bis C4-Alkoxyrest oder einen Phenylrest bedeuten;
X einen verzweigten oder unverzweigten C1-C4-Alkylenrest bedeutet;
R4 einen C1-C4-Alkyl- oder Phenylrest bedeutet;
p und p' unabhängig voneinander eine ganze Zahl bedeuten, die von 1 bis 10 variiert.

10. Verfahren nach einem der vorhergehenden Ansprüche, **dadurch gekennzeichnet, dass** das Verfahren außerdem das Aufbringen einer Pflegezusammensetzung (iv), die von der Pflegezusammensetzung (ii), die das Aminosilikon umfasst, verschieden ist, umfasst, wobei diese Zusammensetzung mindestens eine Silikon- oder Nicht-Silikon-Fettsubstanz umfasst.

## Claims

1. Method for shaping hair fibres comprising the following stages:
(i) application to the hair fibres of a reducing composition comprising one or more cationic polymers, the ratio of the concentration by weight of the reducing agent to the concentration by weight of the cationic polymer being between 0.1 and 10, this application being followed by a rinsing operation,
(ii) application of a care composition, preferably a leave-in care composition, comprising one or more aminated silicones,
(iii) raising the temperature of the hair fibres, using a heating means, to a temperature of between 50 and 280°C, the raising of the temperature being carried out before or after the optional rinsing of the hair fibres,
stage (ii) being carried out after stage (i) and optionally before stage (i),
the method not employing any oxidizing composition.

2. Method according to Claim 1, **characterized in that** the ratio of the concentration by weight of the reducing agent to the concentration by weight of the cationic polymer is between 0.5 and 8 and more preferably between 1 and 5.

3. Method according to either one of Claims 1 and 2, **characterized in that** the cationic polymer used in stage (i) is chosen from cyclopolymers of alkyldiallylamine or of dialkyldiallylammonium, such as the homopolymers or copolymers comprising, as main constituent of the chain, units corresponding to the formulae (V) or (VI): 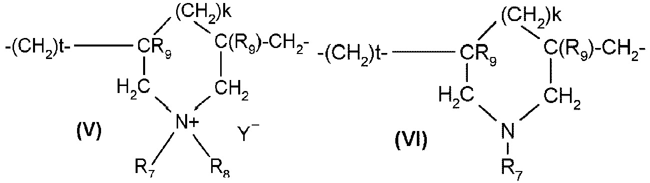 in which formulae:
k and t are equal to 0 or 1, the sum k + t being equal to 1;
R₉ denotes a hydrogen atom or a methyl radical;
R₇ and R₈, independently of one another, denote an alkyl group having from 1 to 6 carbon atoms, a hydroxyalkyl group in which the alkyl group preferably has from 1 to 5 carbon atoms, or a lower (C₁-C₄) amidoalkyl group or else R₇ and R₈ can denote, jointly with the nitrogen atom to which they are attached, heterocyclic groups, such as piperidinyl or morpholinyl; R₇ and R₈,
independently of one another, preferably denote an alkyl group having from 1 to 4 carbon atoms;
Y⁻ is an anion, such as bromide, chloride, acetate, borate, citrate, tartrate, bisulfate, bisulfite,
sulfate or phosphate.

4. Method according to either one of Claims 1 and 2, **characterized in that** the cationic polymer used in stage (i) is chosen from diquaternary ammonium polymers comprising repeat units corresponding to the formula (VII): 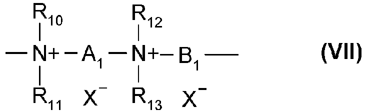 in which formula (VII):
R₁₀, R₁₁, R₁₂ and R₁₃, which are identical or different, represent aliphatic, alicyclic or arylaliphatic radicals comprising from 1 to 6 carbon atoms or lower aliphatic hydroxyalkyl radicals or else R₁₀, R₁₁, R₁₂ and R₁₃, together or separately, constitute, with the nitrogen atoms to which they are attached, heterocycles optionally comprising a second heteroatom other than nitrogen or else R₁₀, R₁₁, R₁₂ and R₁₃ represent a linear or branched C₁-C₆ alkyl radical substituted by a nitrile, ester, acyl, amide or -CO-O-R₁₄-D or -CO-NH-R₁₄-D group, where R₁₄ is an alkylene and D is a quaternary ammonium group;
A₁ and B₁ represent polymethylene groups comprising from 2 to 20 carbon atoms which can be linear or branched and saturated or unsaturated, and which can comprise, bonded to or inserted into the main chain, one or more aromatic rings or one or more oxygen or sulfur atoms or sulfoxide, sulfone, disulfide, amino, alkylamino, hydroxyl, quaternary ammonium, ureido, amide or ester groups, and
X⁻ denotes an anion derived from an inorganic or organic acid;
A₁, R₁₀ and R₁₂ can form, with the two nitrogen atoms to which they are attached, a piperazine ring; in addition, if A₁ denotes a linear or branched, saturated or unsaturated alkylene or hydroxyalkylene radical, B₁ can also denote a group:
-(CH₂)ₙ-CO-D-OC-(CH₂)ₙ-
in which n is between 1 and 100 and preferably between 1 and 50 and D denotes:
a) a glycol residue of formula -O-Z-O-, where Z denotes a linear or branched hydrocarbon radical or a group corresponding to one of the following formulae:
- (CH₂-CH₂-O)ₓ-CH₂-CH₂-
- [CH₂-CH(CH₃)-O]_{y}-CH₂-CH(CH₃)-
where x and y denote an integer from 1 to 4, representing a defined and unique degree of polymerization, or any number from 1 to 4 representing a mean degree of polymerization;
b) a bissecondary diamine residue, such as a piperazine derivative;
c) a bisprimary diamine residue of formula -NH-Y-NH-, where Y denotes a linear or branched hydrocarbon radical or else the divalent radical
-CH₂-CH₂-S-S-CH₂-CH₂-;
d) a ureylene group of formula -NH-CO-NH-.

5. Method according to any one of the preceding claims, **characterized in that** the concentration of reducing agent in the composition of stage (i) is between 0.1% and 5%, with respect to the total weight of the composition.

6. Method according to any one of the preceding claims, **characterized in that** the concentration of cationic polymer(s) in the composition of stage (i) ranges from 0.01 to 10% by weight, preferably from 0.1 to 5% by weight, with respect to the total weight of the composition.

7. Method according to any one of the preceding claims, **characterized in that** the temperature of the hair fibres is raised up to a temperature of between 80°C and 220°C in stage (iii).

8. Method according to any one of the preceding claims, **characterized in that** use is made of a thiol-comprising reducing agent in stage (i).

9. Method according to any one of the preceding claims, **characterized in that** use is made, as aminated silicone in the care composition (ii), of an aminated silicone corresponding to the formula (I): 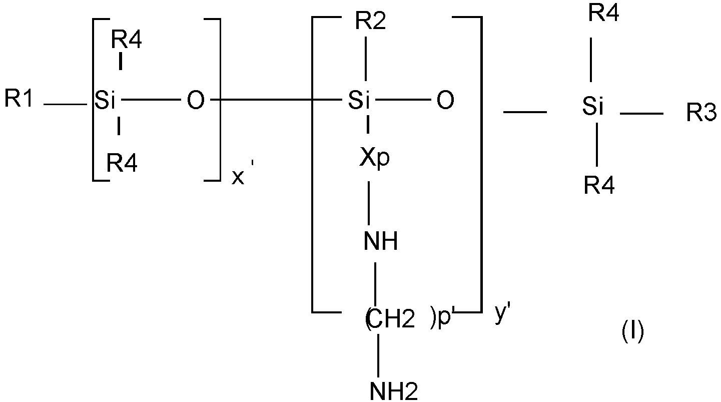
in which x' and y' are integers dependent on the molecular weight, generally such that the said weight-average molecular weight is between 5000 and 500 000 approximately;
R1, R2 and R3, which are identical or different, denote a hydroxyl radical, a C₁ to C₄ alkyl radical, a C₁ to C₄ alkoxy radical or a phenyl radical;
X denotes a branched or unbranched C₁-C₄ alkylene radical;
R4 denotes a C₁-C₄ alkyl radical or a phenyl radical;
p and p' denote, independently of one another, an integer varying from 1 to 10.

10. Method according to any one of the preceding claims, **characterized in that** the method additionally comprises the application of a care composition (iv) different from the care composition (ii) comprising the aminated silicone, this composition comprising at least one silicone or non-silicone fatty substance.
